# EUROPEAN PATENT SPECIFICATION

(11) **EP 1 510 205 B1**
(45) Date of publication and mention of the grant of the patent: **26.03.2008**
(21) Application number: 03019531.7
(22) Date of filing: 01.09.2003
(51) Int. Cl.: A61K 9/00, A61K 9/20, A61K 47/36, A61K 31/196, A61K 31/405, A61K 31/192

(54) **Universal controlled-release composition comprising xanthan gum and sodium alginate**
Universelle Zusammensetzung zur kontrollierten Wirkstofffreigabe enthaltend Xanthangummi und Natriumalginat
Composition universelle à libération retardée comprenant de la gomme de xanthane et de l'alginate de sodium

(43) Date of publication of application: 02.03.2005
(73) Proprietor: JPM - The Jordanian Pharmaceutical Manufacturing Co. Ltd., 11710 Naor (JO)
(72) Inventor: Badwan, Adnan Ali, 11710 Naor (JO); Al-Remawi, Mayyas Mohammad Ahmad, 13713 Russeifa (JO); Salem, Mutaz Bellah A. W. Sheikh, Irbid 22110 (JO)
(74) Representative: Winkler, Andreas Fritz Ernst

(56) References cited:
- EP-A- 0 360 562
- US-A- 5 415 871
- US-A1- 2002 103 181
- US-B1- 6 261 601
- DHOPESHWARKAR V ET AL: "Development of an oral sustained-release antibiotic matrix tablet using in-vitro/in-vivo correlations" DRUG DEVELOPMENT AND INDUSTRIAL PHARMACY 1994 UNITED STATES, vol. 20, no. 11, 1994, pages 1851-1867, XP009025958 ISSN: 0363-9045
- JOHNSON C ET AL: "Release studies from xanthan gum - alginates blends direct compression tablets" PHARMACEUTICAL RESEARCH (NEW YORK), vol. 14, no. 11 SUPPL., November 1997 (1997-11), page S539 XP009025959 Annual Meeting of the American Association of Pharmaceutical Scientists;Boston, Massachusetts, USA; November 2-6, 1997 ISSN: 0724-8741

## Description

The present invention is directed to an universal controlled-release pharmaceutical composition comprising at least one active agent and an inactive matrix, said matrix comprising a mixture of hydrophilic polysaccharide polymers comprising xanthan gum and salts and derivatives thereof, and further comprising sodium alginate and salts and derivatives thereof. The present invention is also directed to the preparation and use of said composition.

### Background of the invention

Over the past years, concepts of controlled drug delivery have been developed extensively, and controlled-release systems designed for this purpose are widely used today. Such systems generally comprise a pharmacologically inactive matrix including one or more active agents which are released from the matrix. Controlling drug delivery may therapeutically be desirable with respect to time (temporal control) and/or place (spatial control).

In case of temporal control, drug release may be sustained or otherwise modified. Alterations in the releasing rates may produce constant drug levels locally at the site of delivery and even constant plasma concentrations. This may result in significantly improved therapeutic effects, e.g. through greater efficacy and/or reduced toxicity. Furthermore, lowering the frequency of administration may lead to a more convenient administration regimen and thus to a better patient's compliance. With respect to temporal control, one focus lies on dermal systems, e.g. plaster. However, systems for oral administration, such as tablets and capsules, are also well known in the art.

Furthermore, oral systems may offer the opportunity to spatially control drug delivery within the gastrointestinal tract. This may be of great advantage, for example, if the intended therapeutic success depends on the portion of the gastrointestinal tract where the pharmacologically active agent should be delivered.

The use of hydrophilic polymers in composing an inactive matrix for controlled release of an active agent are known in the art. For controlled-release solid dosage forms comprising a drug dispersed uniformly in hydrophilic polymers, release of the drug is controlled primarily by diffusion of the drug, or by surface erosion of the hydrophilic polymers in the surrounding medium, or by a combination of the two processes.

In the development of controlled-release pharmaceutical compositions, several polymers, such as hydrophilic polysaccharides, e.g. xanthan gum, sodium alginate and chitosan, have been employed.

### 1. Xanthan gum

Xanthan gum is also known as corn sugar gum. Because of its extraordinary resistance against enzymatic attacks, it is well suitable for oral controlled-release matrix preparations (*cf.* e.g. U.S. 6,261,601).

Xanthan gum has been used alone or in combination with other polymers in controlled-release compositions. U.S. patent No. 4,309,405 used a combination of xanthan gum and hydroxypropylmethyl cellulose (HPMC), hydroxypropyl cellulose and ethyl cellulose in coated tablets for controlled release. These tablets contained 30-72% (by weight) of a mixture of polymers. Drug release from tablets containing xanthan gum was slightly faster in an acidic environment due to more rapid initial surface erosion than at higher pH. After hydration of the gum, drug release was essentially pH-independent (1). The swelling of xanthan gum matrix tablets was reciprocal with salt concentration. Depending on the solubility of the product, the drug release from this matrix was regulated by its swelling behaviour (2). Talukdar *et al.* (3) made a comparison based on compaction and *in vitro* drug release between xanthan gum and HPMC as matrices for controlled release.

### 2. Sodium alginate

Sodium alginate belongs to the group of polyuronic acids and serves as gel forming polymer in controlled-release formulations (*cf.* U.S. patent No. 6,261,601). These methods and their benefits and mechanisms are well documented in the literature (4-12).

The combined use of xanthan gum and sodium alginate has also been described. Patent application WO 02/41876 disclosed a pharmaceutical composition for a controlled-release tablet comprising a β-lactam antibiotic as active agent and a mixture of hydrophilic polymers being selected from the group consisting of sodium alginate and xanthan gum. The composition comprises about 30% to about 90% by weight of active ingredient, about 1% to about 25% by weight of hydrophilic polymers, the latter mixture comprising about 0.1% to about 20% by weight of sodium alginate and about 0.1% to about 20% by weigh of xanthan gum.

U.S. patent No. 6,261,601 (with patent applications WO 00/15198 and EP 1 282 397 derived therefrom) disclosed a pharmaceutical composition in form of a tablet or capsule which provides a combination of temporal and spatial control of drug delivery comprising an active ingredient or drug, a gas generating component, a swelling agent, a viscolysing agent (e.g. xanthan gum), and optionally a gelling polymer (e.g. sodium alginate). A preferred once daily ciprofloxacin formulation comprises 69.9% ciprofloxacin base, 0.34% sodium alginate, 1.03% xanthan gum, 13.7% sodium bicarbonate, 12.1% cross-linked polyvinylpyrrolidone and other pharmaceutical excipients. However, it is also disclosed that the viscolysing agent (e.g. xanthan gum) should be present in an amount from about 0.1% to about 30% and that the gel forming polymer (e.g. sodium alginate) should be present in an amount from about 0.1% to about 20% by weight of the total weight of the composition.

U.S. patent No. 5,415,871 disclosed a sustained-release pharmaceutical formulation comprising an active agent, especially aspirin and other non-steroidal anti-inflammatory drugs, and a sustained-release carrier with xanthan gum. The amount of active agent was 75 to 90% by weight of the formulation in combination with the sustained-release carrier amounting to 10 to 25% by weight. Optionally, a proportion of the xanthan gum may be replaced by one or more additional polymers having sustained-release properties such as sodium alginate. Xanthan gum, however, was preferred to represent a major proportion of the sustained-release carrier.

The patent application EP 0 360 562 disclosed a free-flowing directly compressible slow-release excipient composed of a hydrophilic matrix comprising a heteropolysaccharide (e.g. xanthan gum) and a polysaccharide material (e.g. locust bean gum) capable of cross-linking the heteropolysaccharide. The heteropolysaccharide comprised about 20 to 80% and the polysaccharide material 80 to 20% by weight of the hydrophilic matrix. Other polysaccharide gums (e.g. alginates) may also be added to the hydrophilic material. Chlorpheniramine maleate and propanolol hydrochloride were used in order to demonstrate the sustained-release properties of the excipient.

In Dhopeshwarkar *et al*. (13) sustained-release cephalexin tablets comprising xanthan gum and sodium alginate were disclosed. The concentration range evaluated was 49.3 to 190.7 mg of xanthan gum and 6.9 to 233.1 mg of sodium alginate using a 1,000 mg tablet containing lactose and 750 mg of cephalexin.

Studies on release properties from direct compression tablets containing blends of xanthan gum and sodium alginates were reported by Johnson *et al*. (14). Their results demonstrated that releasing rates of both soluble and insoluble drugs could be altered by varying the xanthan gum/sodium aginate ratio such that an increase of the xanthan gum content in the blends markedly decreased the releasing rates of promethazine hydrochloride and hydrochlorothiazide.

However, formulations for controlled-release compositions are generally developed each for an individual drug. Such a developing procedure involves plenty of time and money. Thus, it would be of great advantage to have a range of universal formulations being suited to provide for a controlled-release behaviour of most active agents.

Thus, there is still a need for the development of controlled-release compositions universally suitable for drugs belonging to only a few groups of a certain classification.

It is therefore an object of the present invention to provide for an universal pharmaceutical composition formulated for the controlled-release of a well defined group of active agents.

Unexpectedly, it turned out that drugs classified as "acidic agents" showed similar controlled-release properties when a polysaccharide mixture of xanthan gum and sodium alginate was used as a matrix.

In particular, it is therefore an object of the present invention to provide for an universal pharmaceutical composition formulated for the controlled-release of acidic active agents.

The controlled-release formulation provided by the present invention has several advantages over the prior art. In particular, the composition is easy and inexpensive to prepare and suited for direct compression. No organic solvents are contained in the composition and the components used are biocompatible. Additionally, the composition has less dose dumping properties.

### Summary of the invention

The present invention provides a controlled-release pharmaceutical composition comprising at least one acidic active agent and an inactive matrix, said matrix comprising a mixture of hydrophilic polysaccharide polymers selected from the group consisting of xanthan gum and salts and derivatives thereof, and further comprising compounds selected from the group consisting of sodium alginate and salts and derivatives thereof, characterised in that the ratio of said acidic active agent and hydrophilic polysaccharide polymer mixture is in the range from about 1:1 to about 1:5, and the ratio of xanthan gum and sodium alginate is in the range of about 1:5 to about 1:15.

In a particularly preferred embodiment of the present invention, the ratio of acidic active agent and hydrophilic polysaccharide polymer mixture is about 1:3.

Preferably, the acidic active agent is selected from the group comprising diclofenac, indomethacin, naproxen and salts and derivatives thereof.

In a preferred embodiment of the present invention, the ratio of xanthan gum and sodium alginate is in the range of about 1:7.5 to about 1:12.5.

In a particularly preferred embodiment of the present invention, the ratio of xanthan gum and sodium alginate is about 1:10.

In just another embodiment of the present invention, the controlled-release composition optionally comprises one or more pharmaceutically acceptable fillers.

In a further aspect, the present invention is directed to a method for preparation of a controlled-release pharmaceutical composition according to the present invention, said method comprising the following steps:
(a) preparing a mixture of hydrophilic polysaccharide polymers selected from the group consisting of xanthan gum and salts and derivatives thereof, and further comprising compounds selected from the group consisting of sodium alginate and salts and derivatives thereof;
(b) mixing said mixture with an acidic active agent, and optionally one or more pharmaceutically acceptable fillers;
(c) processing the preparation into granules;
(d) compacting the granules, preferably into a tablet.

In just another aspect, the present invention is directed to an use of a controlled-release pharmaceutical composition according to the present invention for the manufacture of a medicament for the treatment of an animal, preferably mammal, and most preferably to man.

The inventive composition is easy to manufacture with low cost, provides a homogeneous dispersion, delivers high molecular weight drugs and provides a low possibility of drug-polymer and polymer-polymer interactions.

The term "controlled release" or "controlled delivery" as used in the context of the present invention refers to temporal and/or spatial control. "Temporal" can indicate a "sustained release" and "sustained delivery", respectively, or any release and delivery altered or modulated with respect to time. Temporal "alterations" or "modulations" can result from a comparison with conditions where release or delivery was "uncontrolled", or can mean that alterations or modulations are taking place during time. "Spatial" refers to any localised delivery.

An "active agent" in this context generally means a pharmacologically, therapeutically or otherwise effective agent which may have an effect itself or may be become active e.g. after being metabolised by endogenous enzymes or being converted under certain *in vivo* reaction conditions (e.g. pH).

Illustrative active agents that are useful in the present invention include non-steroidal anti-inflammatory drugs (NSAIDs), such as diclofenac, indomethacin and naproxen. However, many other active agents selected from the groups of any agents orally applied and released into the gastrointestinal tract are also considered, such as anti-infective (e.g. anti-viral, antibacterial, fungicide) agents, anti-inflammatory agents other than NSAIDs, lipid lowering agents, blood pressure affecting agents, immunosuppressants, anti-pyretic agents, analgetics, contraceptives, anti-cancer chemotherapeutics and others.

In general, active agents may be employed as free acids or any pharmaceutically acceptable salts thereof. Derivatives of active agents are also considered.

An "inactive matrix" in this context means those components of the controlled-release pharmaceutical composition which serve as a carrier for the active agent without being itself pharmacologically, therapeutically or otherwise effective. Preferably, the active agent becomes mixed with the inactive matrix components during preparation of the controlled-release pharmaceutical composition such that the active agent is dispersed or otherwise embedded within the inactive matrix.

Apart from xanthan gum, also other hydrophilic polysaccharide polymers belonging to the group of carbohydrate gums such as tragacanth gum, gum karaya, guar gum, acacia gum and the like, and salts and derivatives thereof, may be considered.

Apart from sodium alginate, also other hydrophilic polysaccharide polymers belonging to the group of polyuronic acids such as pectinate and the like, and salts and derivatives thereof, may be considered.

The term "acidic active agent" as used herein refers to an active agent having an excess of acidic functional groups compared to basic functional groups. Examples of acidic functions groups are carboxylic, sulfonic, nitric and phosphoric acid groups, but any other acidic group known in the art is considered. Under basic conditions, an acidic active agent is negatively charged. Acidic functional groups may be present as free acid or as the respective salt depending on conditions known in the art.

Preferably, controlled-release pharmaceutical compositions of the present invention are intended for the preparation of tablets generated by compaction. However, use of the compositions for preparing other solid dosage forms, e.g. capsules, are also considered. Also comprised from the scope of the invention is any refinement of the solid dosage forms, e.g. coating of tablets.

In summary, the present invention provides a formulation of controlled-release pharmaceutical composition which is useful as universal composition for acidic active agents. The compositions comprise a binary mixture of hydrophilic polysaccharides: a combination of xanthan gum and sodium alginate (1:10) is particularly useful for controlled-release of acidic active agents.

In the present invention it is shown that the optimal binary polysaccharide mixture depends on the drug's solubility properties. For drugs having low water solubility, the addition of a single polysaccharide, namely sodium alginate, was found to be sufficient to retard the drug release. Polysaccharide combinations, however, of xanthan gum and sodium alginate were found to be of advantage in controlling the release of water-soluble acidic drugs. For example, the optimum ratio of xanthan gum and sodium alginate was 1:10, when diclofenac sodium was used a an acidic model drug.

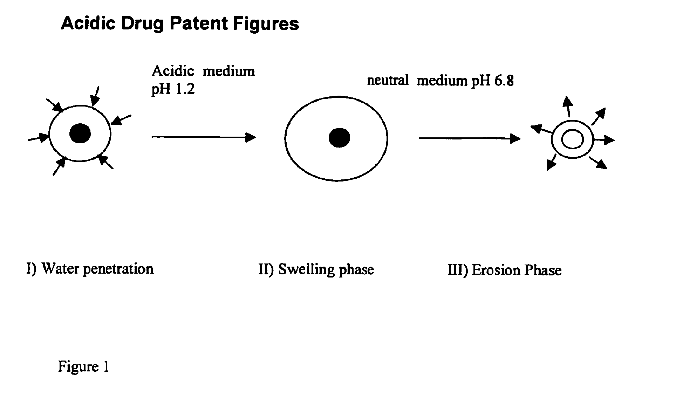

The combination of xanthan gum and sodium alginate (1:10) undergoes a polymer dissolution process occurring at the surface of a tablet. An insoluble coat is formed in the acidic region of the gastrointestinal tract. The polymer erosion and drug dissolution takes place in the duodenal region of the gastrointestinal tract. This mechanism is schematically illustrated in *Figure 1*.

### Detailed description of the invention

The invention shall now be further described and illustrated by the following examples with respect to the attached *Figures 1-6* and *Tables 1-2*. All examples are provided by way of example only, without any intended limitation to the scope of the invention.
**Figure 1** shows a schematic representation of an oral dosage unit, e.g. a tablet, composed of a controlled-release pharmaceutical composition of the present invention.

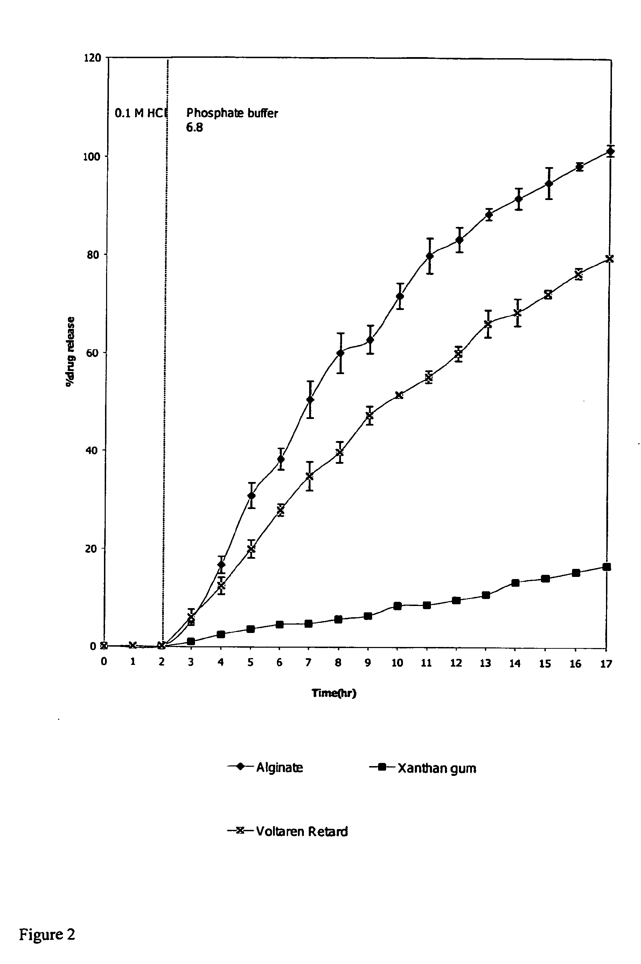

**Figure 2** shows the dissolution profiles of diclofenac sodium (100 mg) from different polymer matrices. The drug to polymer ratio was 1:3. Each formulation was subjected to 0.1 M HCl for 2 hrs and then to phosphate buffer, pH 6.8 for the rest of the dissolution period. Dissolution conditions: RPM, 50; apparatus, USP Paddle; volume, 600 ml; temperature, 37°C. Each point represents the mean of n=3. Error bars represent the standard deviation.

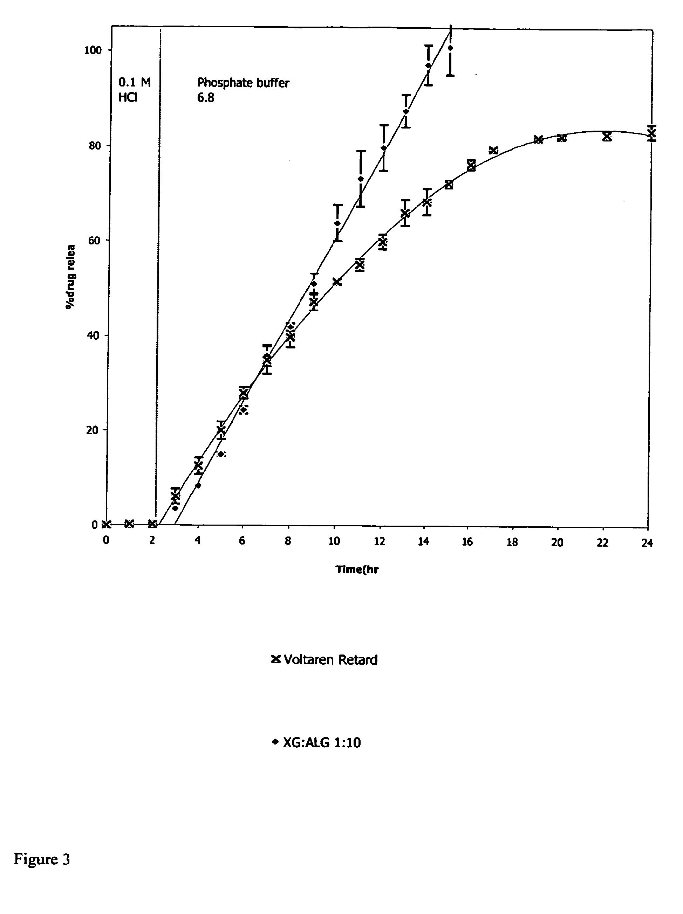

**Figure 3** shows the release profiles of diclofenac sodium (100 mg) from a matrix tablet comprising the polymer mixture of xanthan gum and sodium alginate (1:10) compared to those from Voltaren® tablets. Dissolution conditions: 0.1 M HCl for 2 hrs, then phosphate buffer, pH 6.8; RPM, 50; apparatus, USP Paddle; volume: 600 ml; temperature, 37°C. Each point represents the mean of n=3. Error bars represent the standard deviation

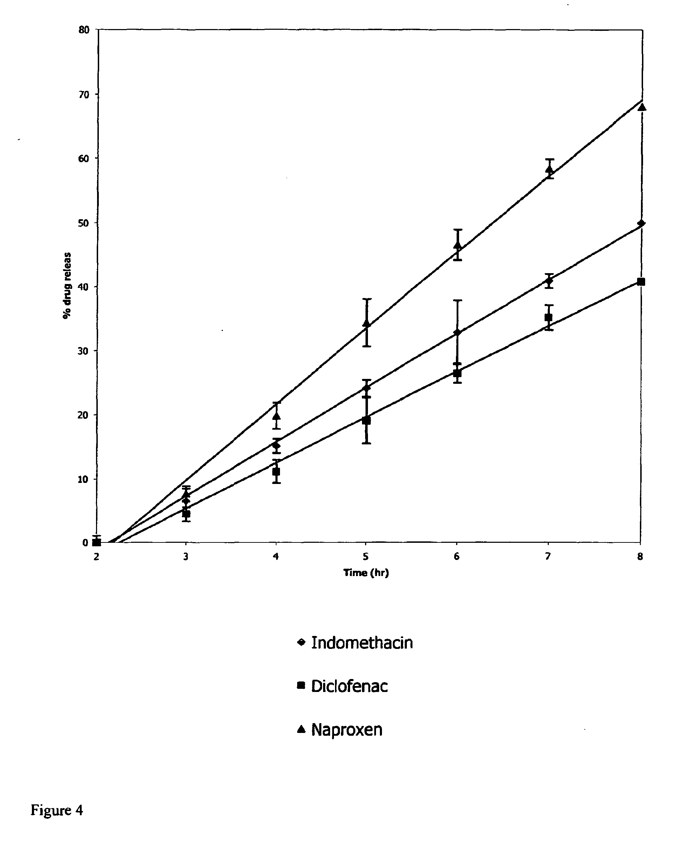

**Figure 4** shows the dissolution profiles of acidic drugs from matrices comprising xanthan gum and sodium alginate (1:10). First, 2 hrs in 0.1 M HCl, then in phosphate buffer, pH 6.8 for the rest. Dissolution conditions: RPM, 75; apparatus, USP basket; volume, 750 ml; temperature, 37°C. Each point is the average of three readings. Error bars represent the standard deviation.

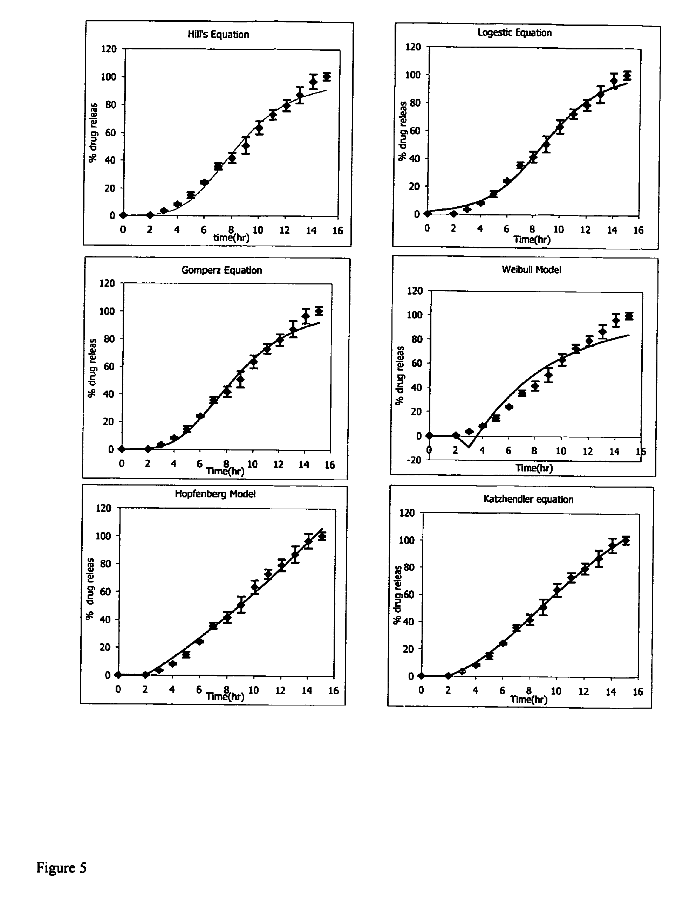

**Figure 5** shows models used to simulate the dissolution profile of diclofenac sodium from a polymer mixture comprising xanthan gum and alginate (1:10). D:P ratio was 1:3. Dissolution conditions: RPM, 50; apparatus, USP Paddle; volume, 600 ml; 0.1 M HCl, then phosphate buffer, pH 6.8; temperature, 37°C. Dots refer to the experimental data and the line represents model simulation.

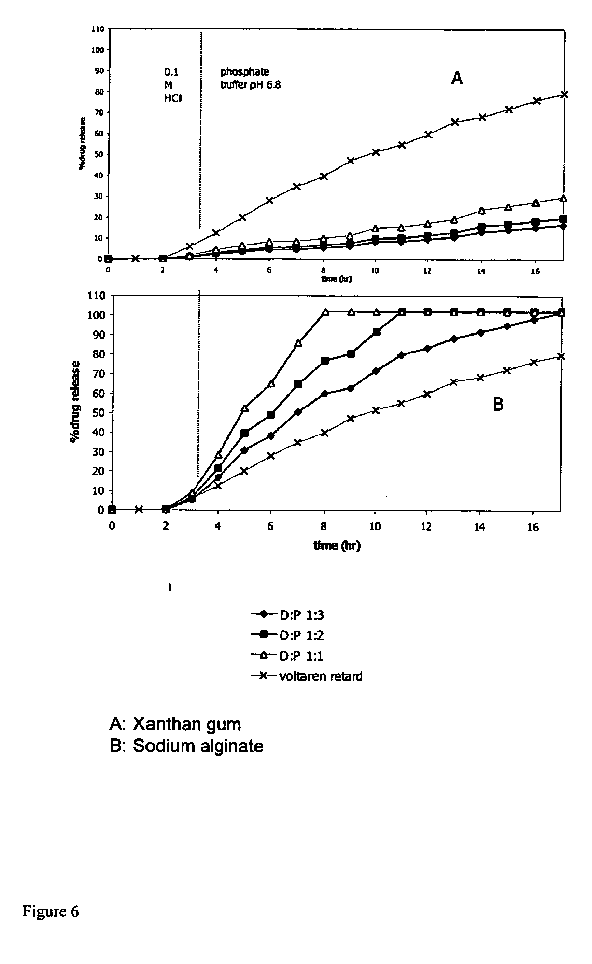

**Figure 6** shows the dissolution profiles of diclofenac sodium (100 mg) from xanthan gum (A) and sodium alginate (b) matrices using different drug to polymer (D:P) ratios. Each formulation was subjected to 0.1 M HCl for 2 hrs and then to phosphate buffer, pH 6.8 for the rest of the dissolution period. Dissolution conditions: RPM, 50; apparatus, USP Paddle; volume, 600 ml; temperature, 37°C.

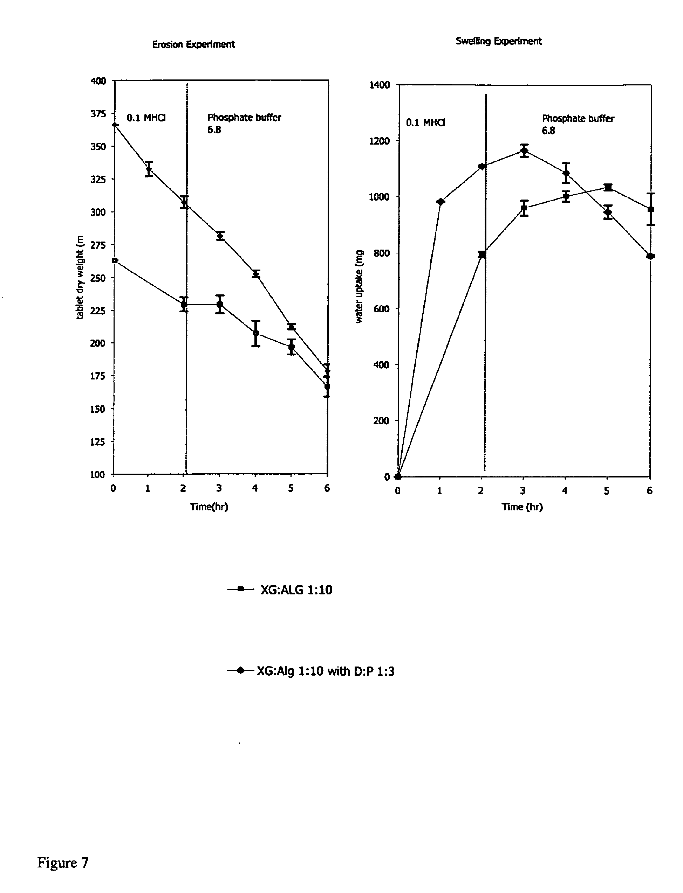

**Figure 7** shows the erosion and water absorption curves of a matrix of xanthan and sodium alginate (1:10) with diclofenac sodium ("real matrix") and without diclofenac sodium ("placebo matrix").

### Example 1: Preparation of controlled-release tablets and compaction studies

In order to assess the controlled-release properties and the matrix-forming ability of the hydrophilic polysaccharides which combination is object of the present invention, overall compaction studies and *in vitro* drug releasing tests were conducted.

Pharmacologically active agents which were used as model acidic agents in the present invention were diclofenac, indomethacin, and naproxen.

### 1. Preparation of controlled-release tablets

Polymers finally used were anionic xanthan gum and anionic sodium alginate (in a first approach, also cationic chitosan was tested). Drug to polymer ratio was set constant at 1:3 (*cf. Table 1* below), irrespective of whether the polysaccharides were individually used or a binary polymer mixture was used instead. In the latter case, the ratios of xanthan gum and sodium alignate tested first were 1:4, 1:1 and 4:1, then other ratios were used for optimisation (*cf. Table 1* below). Finally, a ratio of 1:10 turned out to be optimal.

**Table 1: Summary of formulations used in the present invention**

| Model acidic drug | Active ingredient per tablet [mg] | Tablet weight [mg] | D:P ratio* polymer | Polymers ratio |
|---|---|---|---|---|
| Diclofenac sodium (water-soluble) | 100 | 400 | 1:3 | CHALG, XG single XG:ALG 1:1, 1:4,4:1, 1:6, 1:8, 1:10, 1:20, 1:30 |
| | | | | |
| Diclofenac (water-insoluble) | 100 | 400 | 1:3 | CHALG, XG single XG:ALG 1:1, 1:4, 4:1, 1:10 |
| | | | | |
| Indomethacin (water-insoluble) | 75 | 300 | 1:3 | XG:ALG 1:10 |
| | | | | |
| Naproxen (water-insoluble) | 100 | 400 | 1:3 | XG:ALG 1:10 |

| | | | | |
|---|---|---|---|---|
| CH, chitosan; ALG, sodium alginate; XG, xanthan gum. * Drug to single polysaccharide ratio or drug to binary polysaccharides ratio (by weight) | | | | |

Polysaccharide mixtures comprising the model acidic agents were prepared in form of powders and granules (*cf.* below), and the mechanical properties were evaluated. The strength of tablets prepared from powder formulations was higher than that prepared from granules. However, no differences in drug release were observed.

The difference in tablet strength became obvious during compaction. Generally, granules possess better flow properties (are more flowable) than powder formulations but show larger elastic recovery stress upon compression. In the present invention, it was found that the ordinary granulation method resulted in the formation of porous granules. Such pores will contract air pockets upon compaction. These air pockets resulted in a decrease of tablet strength and in an increase in elastic recovery. Formation of small beads (granules) through modem technology may be the most appropriate solution. This would bring non-porous particles resulting in compaction with less elastic recovery and allowing easier handling by reducing the pressure applied in the tablet machine.

### 1.1. Tablet preparation from powder components

A model acidic agent (*cf. Table 1*) was mixed with a single polysaccharide (xanthan gum or sodium alginate) and with a binary polysaccharide mixture of xanthan gum/sodium alginate, respectively. Prior to mixing, powders were deagglomerated by sieving. The components of each preparation were geometrically mixed by porcelain mortar and pestle for about 10 minutes before compression. Biplanar, cylindric tablets were manufactured by compressing the powder mixtures applying a pressure of about 443 MPa for 15 seconds by a low speed compaction machine (hydraulic KBr press or an Instron Instrument). Tablets were sealed properly with aluminum foil and placed in amber glass bottles for 24 hours.

The weight of the compressed mixture was the determinant in the selection of the diameter. In previous studies, the diameter used was optimised to 13 mm to compress weights of 300-400 mg tablet weight.

### 1.2. Preparation of tablets from granules

The optimal formulation obtained with tablets prepared from powder components, namely xanthan gum/sodium alginate at a ratio of 1:10 and a drug to polymer ratio of 1:3, was used to prepare tablet starting material in granule form comprising diclofenac sodium.

100 g powder mixture as described above (*cf. 1.1.*) was granulated with a 1:1 mixture of ethanol and water until a granulation wet mass was obtained (~ 60 ml). The wet mass was forced through a sieve, mesh No. 20, and then dried at 65°C until a moisture content was detected similar to the corresponding powder formula using a Karl Fisher titrator. The dried granules were passed through a sieve, aperture size 500 µm. Samples were stored in well-closed bottles at room temperature. These granular mixtures were characterised before and after compression into tablet dosage forms. Biplanar, cylindrical tablets were manufactured by compressing the granules applying a pressure of about 443 MPa for 15 seconds by a hydraulic KBr press. The die diameter used was optimised to 13 mm to compress 300-400mg.

### Example 2: Releasing in vitro assays with acidic drugs

There are many published *in vitro* dissolution tests for controlled-release delivery systems. These include the use of USP Paddle or Basket apparatuses. Dissolution media used was an acidic medium in the first two hours followed by a basic medium for the rest of the experimental period (15).

### 1. Evaluation of optimal polymer ratios

In the development of a formulation for controlled-release of an acidic active agent, suitable ratios of drug to polymers (D:P) were evaluated first. Diclofenac sodium used as a model acidic drug was mixed with xanthan gum, sodium alginate and chitosan each separately with the following D:P ratios: 1:1, 1:2 and 1:3. Diclofenac releasing rates were determined. Voltaren^{®} retard, a diclofenac sodium prolonged-release tablet preparation commercially available from Novartis (Switzerland) was used for comparison. Xanthan gum and chitosan resulted in high drug release at a D:P ratio of 1:1 and 1:2, and retardation was the best at a D:P ratio of 1:3 (Fig. 6). A comparison of the diclofenac releasing rates exhibited by xanthan gum and sodium alginate taking Volatren^{®} retard as a reference using a D:P ratio of 1:3 is shown in *Fig. 2*. There, it is demonstrated that diclofenac sodium release is retarded with xanthan gum and chitosan compared to Voltaren^{®} retard whereas diclofenac sodium release retardation is even least of all with sodium alginate.

However, upon combining xanthan gum and sodium alginate, a significant drug release was observed, suggesting that an optimal balance between drug release and release retardation was achievable by using this mixture. Thus, optimisation of the ratio was carried out leading to the selection of a xanthan gum/sodium alginate ratio of 1:10 as the best formulation. *Fig. 3* demonstrates that diclofenac sodium release from Voltaren^{®} retard levels off at times when the release from xanthan gum/alginate (1:10) mixture is still linear. Similar results were obtained with indomethacin and naproxen (*Fig. 4*). Linearity of the curve happened in the total period of dissolution of diclofenac sodium, i.e. until 100% drug release is achieved after 16 hours of dissolution in our matrix. But this did not occur for Voltaren^{®} retard which reached about 70% release as linear then a plateau was developed in the next region. The used of diclofenac, indomethacin, and naproxen resulted in a similar behaviour if data were shown for the total period of dissolution (24 hours).

### 2. Mechanism of controlled-release

The mechanism of controlled-release resulted from a polymer erosion process. Using different established models, the model of Kazhendler (16) turned out to have the best curve fitting (*cf.* *Fig. 5*) resulting in the highest model selection criterion (MSC) (*cf. Table 2* below). This model assumes polymer erosion to be the controllable mechanism for drug release.

The results from the different models applied are shown in *Fig. 5*. Hill's equation, Weibull equation, Gomperz equation, Logestic equation, Hopfenberg equation and Katzhendler equation were adapted to the observed data using non-linear least squares curve fitting (*Fig. 5*) (16-19). Katzhendler and Hopfenberg gave the largest MSC and lowest sum of squared residues (*cf. Table 2* below). Also the model parameters estimated from these two models were much closer to the observed result (*cf. Table 2* below) indicating the superiority of them to describe the drug release from an erodible matrix system.

**Table 2: Models used to describe the mechanism of diclofenac sodium release from a matrix of xanthan gum:sodium alginate with 1:10 ratio.**

| Model | MSC | ΣRes*d*²/(*n*-2) |
|---|---|---|
| Hill | 3.92 | 21.50 |
| Hopfenberg | 4.74 | 9.50 |
| Katzhendler | 6.15 | 2.32 |
| Weibull | 2.43 | 83.67 |
| Gomperz | 2.40 | 16.28 |
| Logestic | 4.70 | 9.92 |

Hopfenberg has described the mechanism of release from simple erodible slab, cylinders and spheres. According to his model, n is the shape factor, 3 for sphere, 2 for a cylinder and 1 for a slab (16). The *n* value in our matrix system was found to be 1.28. This value would be for a matrix shape that behaves like a slab and a cylinder at the same time. This is justified since the matrix diameter was much larger (about 6.5 times) than the matrix thickness.

The advantage of Katzhendler equation over Hopfenberg equation is that Katzhendler suggests that the matrix erosion occurs in both radial and vertical directions (17). This led to better data fitting of Katzhendler equation as indicated by higher MSC. However, the erosion rate in the radial direction (kₐ) was larger than that in the vertical direction (k_{b}). Thus, according to these models, polymer erosion is the main mechanism of drug release.

The release of diclofenac sodium from xanthan gum/sodium alginate (1:10) matrix occurred largely during the dissolution of the hydrophilic polymer. This was confirmed by conducting a swelling/erosion study of two matrices *(**Fig.* 7): first, a matrix containing the polymer mixture (xanthan gum and sodium alginate 1:10) without drug, called placebo matrix, and second, a matrix containing the same polymer mixture (xanthan gum and sodium alginate 1:10) and diclofenac with D:P ratio 1:3, called real matrix. Both the placebo and real matrices showed a decrease in the tablet dry weight with time indicating that an erosion mechanism would be responsible for drug release (*cf.* *Fig. 7*, left panel). However, the real matrix showed a steeper slope which means a higher tablet erosion rate of real matrix than the placebo matrix, especially in the phosphate buffer phase. The consequences of erosion in phosphate buffer would be as follows. When the solvent wets the polymers at the surface, they swell (as demonstrated in *Fig. 7*, right panel) and their swelling will delay further solvent penetration to the inside of the matrix. On the other hand, the drug unlike the hydrophilic polymers would not swell and instead dissolves in the neutral pH. This leads to the production of a more porous system in the real matrix that contains the drug. This should enhance solvent penetration inside the matrix. Consequently, erosion rate would be higher from the real matrix. In consequence, this experiment further supported the previous assumption that drug release is mediated by a polymer erosion process.

The release of diclofenac sodium from the xanthan gum/sodium alginate (1:10) matrix was characterised as follows. Drug release occurred upon exposure of the matrix to phosphate buffer, pH 6,8 following two hours in the acidic dissolution phase (t_{lag}) (*cf.* *Fig. 3*). Polymers within the matrix started to dissolve in the nearly neutral medium. The drug was released slowly from the matrix system; it took about 14 hours for the drug to complete its release. The release followed a zero-order kinetic in the nearly neutral medium. The cylindrical tablet initial diameter and thickness were about 1.3 and 0.2 cm, respectively. It is believed that some of the diclofenac sodium, a water soluble drug, would be released by diffusion, but this amount might be much smaller than the amount released by matrix erosion.

Since the drug release is controlled by the rate of polymer erosion, the use of xanthan gum and sodium alginate combinations as erodible matrix has several advantages in addition to all other advantages of matrix dissolution systems. As demonstrated in *Fig. 3*, a zero-order delivery is possible to maintain over a prolonged time-period. Any alteration of the polymer ratio will change the slope of release but will still keep a zero-order pattern. Furthermore, this xanthan gum-sodium alginate matrix is easy to manufacture with low costs. A homogenous dispersion of the combined compounds can be achieved. Also high molecular weight drugs can be delivered, and there is only a low possibility of drug-polymer and polymer-polymer interactions.

The features disclosed in the foregoing description, in the claims and/or in the drawings may, both separately and in any combination thereof, be material for realising the invention in diverse forms thereof.

### References

No.
1 V. Dhopeshwarkar and J. Zataz. Evaluation of Xanthan gum in the Preparation of Sustained Release Matrix Systems. Drug Dev. Ind. Pharm., 19, pp 999-1017, 1993.
2 M. Talukdar, R. Kinget. Swelling and Drug Release Behavior of Xanthan gum Matrix Tablet. Int. J. Pharm., 120, pp 63-72, 1995.
3 M. Talukdar., A. Michoel, P. Rombaut, R. Kinget. Comparative Study on Xanthan gum and HPMC as Matrices for Controlled Release Drug Delivery 1-Compaction and In Vitro drug release Behavior. Int. J. Pharm., 129, pp 233-241, 1996.
4 S. Sugawara, T. Imai ,and M. Otagiri, The Controlled Release of Prednisolone Using Alginate Gel. Pharm. Res., 11, pp 272-277, 1994.
5 S. Lin, J. Ayres,. Calcium Alginate Beads as Core Carriers of 5-Aminosalisalicylic Acid. Pharm. Res., 9, pp1128-1131, 1992.
6 C. Kook K. Lee. The Controlled Release of Blue Dextran from alginate Beads Int. J. Pharm., 79, pp 11-19, 1992.
7 S. Morgan, A. Al-Shamakhani, D. Callant, E. Schacht, J. Woodley, R. Duncan. Alginate as Drug Carriers: Covalent Attachment of Alginates to Therapeutic Agents Containing Primary Amine Groups. Int.J. Pharm., 122, pp 1221-128, 1995.
8 P. Giunchedi, E. Gavini, M. Moretti, G. Pirisino. Abstract: Evaluation of Alginate Compressed Matrices as Prolonged Drug Delivery Systems. AAPS Pharmsciech., 1,2000.
9 H.R Bhagat, R. Mendes, E. Mathiowitz, and H. Bhargava. Kinetics and Mechanism of Release from Calcium Alginate membrane Coated Tablets. Drug Dev. Ind. Pharm.. 20, pp 387-394, 1994.
10 L. Tay, L. Khoh, C. Loh, and E. Khor. Alginate-Chitosan Coacervateation in Production of Artificil Seeds. Biotech. Bioeng., pp 42, 449-454, 1993.
11 H. Takeuchi, T. Yasuji, H. Yamamoto and Y. Kawashima. Spray Dried Lactose Composite Particles Containing an Ion Complex of Alginate -Chitosan for Designing a Dry-Coated Tablet Having a Time Controlled Releasing Function. Pharm. Res., 17, pp 94-99, 2000.
12 O. Gaserod, O. Smidsrod., G. Skjak. Microencapsules of Alginate-Chitosan -I Aquantitative Study of the interaction between alginate and Chitosan. Biomaterials, 19, pp 1815-1825, 1998
13 V. Dhopeshwarkar J.C. O'Keeffe, J.L. Zatz. R. Deeter. M. Horton Development of an oral sustained-release antibiotic matrix tablet usine in-vitro/in-vivo correlations. Drug. Dev. Ind. Pharm. 20. pp. 1851-1867, 1994.
14 C. Johnson, G. Colegrove. T. Nivaggioli. Release studies from xanthan-alginates blends direct compression tablets Phann. Res 14. Suppl. 1997-11, p. S-539. 1997.
15 A. Nokhodchi, Dj. Farid, M. Najafi, M. Adrangui. Studies on Controlled Release Formulations of Diclofenac Sodium. Drug Dev. Ind. Pharm., 23, pp1019-1023, 1997.
16 I. Katzhendeler, A. Hoffman, A. Goldberger. Modeling of drug Release from Erodible Tablets. J.Pharm.Sci. 86, pp110-115, 1997.
17 J. Cobby, M. Mayersohn, G. Walker. Influence of Shape Factors on Kinetics of Drug Release from Matrix Tablets I: Theoretical. J. Pharm. Sci, 63, pp 725-737, 1974.
18 M. Labastie, R. Nacco, J. Cumps. Tablet Dissolution Parameters: a statistical evalution. J.Pharm. Biomed. Anal., 10, pp 1105-1108, 1993.
19 T. Koizumi, G. Ritthidej, T. Phaechamud. Mechanistic Modeling of Drug Release from Chitosan coated tablets. J.Controlled Release, 70, pp 277-284, 2001.

## Claims

1. A controlled-release pharmaceutical composition comprising at least one acidic active agent and an inactive matrix, said matrix comprising a mixture of hydrophilic polysaccharide polymers, said mixture consisting of xanthan gum and sodium alginate, **characterised in that** the ratio of said acidic active agent and said hydrophilic polysaccharide polymer mixture is in the range from about 1:1 to about 1:5, and the ratio of xanthan gum and sodium alginate is in the range of about 1:5 to about 1:15.

2. The controlled-release pharmaceutical composition according to claim 1, wherein the ratio of acidic active agent and polymer mixture is about 1:3.

3. The controlled-release pharmaceutical composition according to claim 1 or 2, wherein the acidic active agent is selected from the group comprising diclofenac, indomethacin, naproxen, and salts and derivatives thereof.

4. The controlled-release pharmaceutical composition according to any of the preceding claims, wherein the ratio of xanthan gum and sodium alginate is in the range of about 1:7.5 to about 1:12.5.

5. The controlled-release pharmaceutical composition according to any of the preceding claims, wherein the ratio of xanthan gum and sodium alginate is about 1:10.

6. The controlled-release pharmaceutical composition according to any of the preceding claims, optionally comprising one or more pharmaceutically acceptable fillers.

7. A method for preparation of a controlled-release pharmaceutical composition according to any of the preceding claims, said method comprising the following steps:
(a) preparing a mixture of hydrophilic polysaccharide polymers consisting of xanthan gum and sodium alginate, wherein the ratio of xanthan gum and sodium alginate is in the range of about 1:5 to about 1:15.
(b) mixing said mixture with an acidic active agent, and optionally one or more pharmaceutically acceptable fillers;
(c) processing the preparation into granules;
(d) compacting the granules, preferably into a tablet.

8. Use of a controlled-release pharmaceutical composition according to any of the preceding claims 1 to 6 for the manufacture of a medicament for the treatment of an animal, preferably a mammal, and most preferably man.

## Patentansprüche

1. Pharmazeutische Zusammensetzung zur kontrollierten Freigabe, umfassend wenigstens ein saures aktives Agens und eine inaktive Matrix, wobei die Matrix eine Mischung aus hydrophilen Polysaccharidpolymeren umfaßt, wobei die Mischung aus Xanthangummi und Natriumalginat besteht, **dadurch gekennzeichnet, daß** das Verhältnis des sauren aktiven Agens und der hydrophilen Polysaccharidpolymermischung im Bereich von etwa 1:1 1 bis etwa 1:5 liegt, und daß das Verhältnis von Xanthangummi und Natriumalginat im Bereich von etwa 1:5 bis etwa 1:15 liegt.

2. Pharmazeutische Zusammensetzung zur kontrollierten Freigabe nach Anspruch 1, wobei das Verhältnis des sauren aktiven Agens und der Polymermischung etwa 1:3 ist.

3. Pharmazeutische Zusammensetzung zur kontrollierten Freigabe nach Anspruch 1 oder 2, wobei das saure aktive Agens ausgewählt ist aus der Gruppe umfassend Diclofenac, Indomethacin, Naproxen und Salze und Derivate derselben.

4. Pharmazeutische Zusammensetzung zur kontrollierten Freigabe nach einem der vorangehenden Ansprüche, wobei das Verhältnis von Xanthangummi und Natriumalginat im Bereich von etwa 1:7,5 bis etwa 1:12,5 ist.

5. Pharmazeutische Zusammensetzung zur kontrollierten Freigabe nach einem der vorangehenden Ansprüche, wobei das Verhältnis von Xanthangummi und Natriumalginat etwa 1:10 ist.

6. Pharmazeutische Zusammensetzung zur kontrollierten Freigabe nach einem der vorangehenden Ansprüche, welche optional einen oder mehrere pharmazeutisch annehmbare Füllstoffe umfaßt.

7. Verfahren zum Herstellen einer pharmazeutischen Zusammensetzung zur kontrollierten Freigabe nach einem der vorangehenden Ansprüche, wobei das Verfahren die folgenden Schritte umfaßt:
(a) Herstellen einer Mischung eines hydrophilen Polysaccharidpolymers bestehend aus Xanthangummi und Natriumalginat, wobei das Verhältnis von Xanthangummi und Natriumalginat im Bereich von etwa 1:5 bis etwa 1:15 ist,
(b) Mischen der Mischung mit einem sauren aktiven Agens und optional einem oder mehreren, pharmazeutisch annehmbaren Füllstoffen;
(c) Verarbeiten der Zubereitung in Granalien;
(d) Kompaktieren der Granalien, bevorzugt in eine Tablette.

8. Verwendung einer pharmazeutischen Zusammensetzung zur kontrollierten Freigabe nach einem der vorangehenden Ansprüche 1 bis 6 für die Herstellung eines Medikaments für die Behandlung eines Tieres, bevorzugt eines Säugetieres und am bevorzugtesten eines Menschen.

## Revendications

1. Composition pharmaceutique à libération retardée comprenant au moins un agent actif acide et une matrice inactive, ladite matrice comprenant un mélange de polymères de polysaccharide hydrophile, ledit mélange étant constitué de gomme de xanthane et d'alginate de sodium, **caractérisée en ce que** le rapport entre ledit agent actif acide et ledit mélange de polymères de polysaccharide hydrophile se trouve dans la plage d'environ 1:1 à environ 1:5, et le rapport entre la gomme de xanthane et l'alginate de sodium se trouve dans la plage d'environ 1:5 à environ 1:15.

2. Composition pharmaceutique à libération retardée selon la revendication 1, dans laquelle le rapport entre l'agent actif acide et le mélange de polymères est d'environ 1:3.

3. Composition pharmaceutique à libération retardée selon la revendication 1 ou 2, dans laquelle l'agent actif acide est choisi dans le groupe comprenant le diclofénac, l'indométhacine, le naproxène, et les sels et dérivés de ceux-ci.

4. Composition pharmaceutique à libération retardée selon l'une quelconque des revendications précédentes, dans laquelle le rapport entre la gomme de xanthane et l'alginate de sodium se trouve dans la plage d'environ 1:7,5 à environ 1:12,5.

5. Composition pharmaceutique à libération retardée selon l'une quelconque des revendications précédentes, dans laquelle le rapport entre la gomme de xanthane et l'alginate de sodium est d'environ 1:10.

6. Composition pharmaceutique à libération retardée selon l'une quelconque des revendications précédentes, comprenant de manière facultative une ou plusieurs charges acceptables sur le plan pharmaceutique.

7. Procédé de préparation d'une composition pharmaceutique à libération retardée selon l'une quelconque des revendications précédentes, ledit procédé comprenant les étapes suivantes :
(a) la préparation d'un mélange de polymères de polysaccharide hydrophile constitué de gomme de xanthane et d'alginate de sodium, dans lequel le rapport entre la gomme de xanthane et l'alginate de sodium se trouve dans la plage d'environ 1:5 à environ 1:15.
(b) le mélange dudit mélange avec un agent actif acide, et de manière facultative une ou plusieurs charges acceptables sur le plan pharmaceutique ;
(c) le traitement de la préparation en granules ;
(d) la compaction des granules, de préférence en un comprimé.

8. Utilisation d'une composition pharmaceutique à libération retardée selon l'une quelconque des revendications 1 à 6 précédentes pour la fabrication d'un médicament pour le traitement d'un animal, de préférence un mammifère, et de manière préférée entre toutes un homme.
